# EUROPEAN PATENT SPECIFICATION

(11) **EP 0 316 128 B1**
(45) Date of publication and mention of the grant of the patent: **03.03.1993**
(21) Application number: 88310465.5
(22) Date of filing: 07.11.1988
(51) Int. Cl.: C07H 17/00, A61K 31/70

(54) **Antibacterial 9-deoxo-9a-allyl and propargyl-9a-aza-9a-homoerythromycin A derivatives**
Antibakterielle 9-Deoxy-9a-allyl und Propargyl-9a-aza-9a-homoerythromycin-A-Derivate
Dérivés à activité antibactérienne 9-déoxo-9a-allyl et propargyl-9a-aza-9a-homoérythromycine A

(30) Priority: 10.11.1987 WO PCT/US87/02938
(43) Date of publication of application: 17.05.1989
(73) Proprietor: PFIZER INC., New York, N.Y. 10017 (US)
(72) Inventor: Bright, Gene Michael, Groton, CT (US)
(74) Representative: Wood, David John

(56) References cited:
- EP-A- 0 101 186
- EP-A- 0 132 944
- THE JOURNAL OF ANTIBIOTICS, vol. XLI, no. 8, August 1988, Japan Antibiotics Research Association; G.M.BRIGHT et al.: "Synthesis, in vitro and vivo activity of novel 9-deoxo-9a-aza-9a-homoerythromycin a derivatives; a new class of macrolide antibiotics, the azalides" pages 1029-1047
- ABSTRACT of 27th Interscience Conference on Antimicrobial Agents and Chemotherapy, no. 238, 0ctober 1987

## Description

This invention relates to novel derivatives of 9-deoxo-9a-aza-9a-homoerythromycin A. More particularly it relates to 9a-allyl derivatives of 9-deoxo-9a-aza-9a-homoerythromycin A, to pharmaceutically acceptable acid addition salts thereof and the use of said compounds as antibacterial agents.

Erythromycin A is a macrolide antibiotic produced by fermentation and described in U.S. Patent No. 2,653,899. Numerous derivatives of erythromycin A have been prepared in efforts to modify its biological and/or pharmacodynamic properties. Erythromycin A esters with mono- and dicarboxylic acids are reported in Antibiotics Annual, 1953-1954, Proc. Symposium Antibiotics (Washington, D.C.), pages 500-513 and 514-521, respectively. U.S. Patent No. 3,417,077 describes the cyclic carbonate ester of erythromycin A, the reaction product of erythromycin A and ethylene carbonate, as an active antibacterial agent.

U.S. Patent 4,328,334, issued May 4, 1982 describes 9-deoxo-9a-aza-9a-homoerythromycin A and refers to it by the came 11-aza-10-deoxo-10-dihydroerythromycn A. Since said compound is a ring expanded (homo) derivative of erythromycin A, nitrogen (aza) being the additional atom of the ring system, the nomenclature 9-deoxo-9a-aza-9a-homoerythromycin A is preferred for the parent ring system of the compounds of this invention.

Belgian Patent 892,357, published July 1, 1982, and its British counterpart, Application 2,094,293A, published September 15, 1982, disclose the N-methyl derivative of 9-deoxo-9a-aza-9a-homoerythromycin A, while U.S. Patent 4,526,889 claims the corresponding 4''-epi-9a-methyl isomer. U.S. Patent 4,512,982 claims 9-deoxo-9a-methyl-9a-aza-9a-homoerythromycin A derivatives wherein the substituent at the 4'' can be hydrogen or amino.

U.S. Patent 4,382,085, issued May 3, 1983 describes 4''-epi erythromycin A; i.e., the 4''-OH group has the axial configuration. The 4''-OH in erythromycin A has the equatorial configuration.

9-Deoxo-9a-propargyl-9a-aza-9a-homoerythromycin A was disclosed in the Abstract of 27th Interscience Conference on Antimicrobial Agents and Chemotherapy (October, 1987) as an HPLC standard in tissue analysis of azithromycin and analogs thereof.

It has now been found that 9a-allyl derivatives of 9-deoxo-9a-aza-9a-homoerythromycin A are effective antibacterial agents against Gram-positive and Gram-negative bacteria. The compounds have the formula (I) 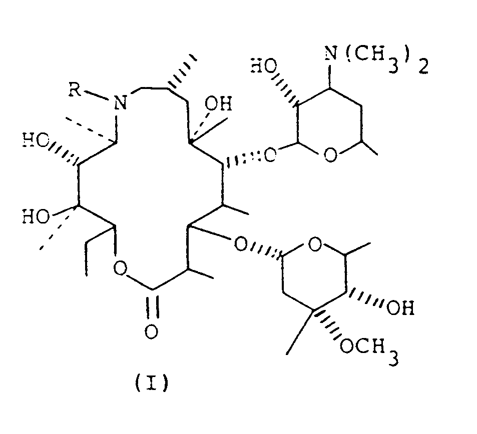
wherein R is allyl.

Also included in this invention, and useful for the same purpose as formula (I) compounds, are the pharmaceutically acceptable acid addition salts thereof. Included among said salts, but by no means limited to said salts, are those enumerated below: hydrochloride, hydrobromide, sulfate, phosphate, formate, acetate, propionate, butyrate, citrate, glycolate, lactate, tartrate, malate, maleate, fumarate, gluconate, stearate, mandelate, pamoate, benzoate, succinate, p-toluenesulfonate and asparate.

The compounds of the present invention are useful in a method for treating a bacterial infection in a mammal which comprises administering to said mammal an antibacterially effective amount of a compound of formula (I). The invention also includes a pharmaceutical composition, preferably in unit dosage form, for treating a bacterial infection, which comprises an antibacterially effective amount of a compound of formula (I) and a pharmaceutically acceptable carrier or diluent. Additionally, compounds of formula (I) exhibit significant activity against Neisseria gonorrhea and Haemophilus in vitro and against many Gram-positive and Gram-negative microorganisms in vivo. In their useful oral activity and unexpectedly long serum half-life in mammals, the formula (I) compounds are like 9-deoxo-9a-methyl-9a-aza-9a-homoerythromycin A, and quite unlike the corresponding 9a-desmethyl compound 9-deoxo-9a-aza-9a-homoerythromycin A which exhibits no practical oral activity in vivo, and a substantially shorter serum half-life.

The compounds of formula (I) wherein R is as previously defined are prepared according to the following scheme: 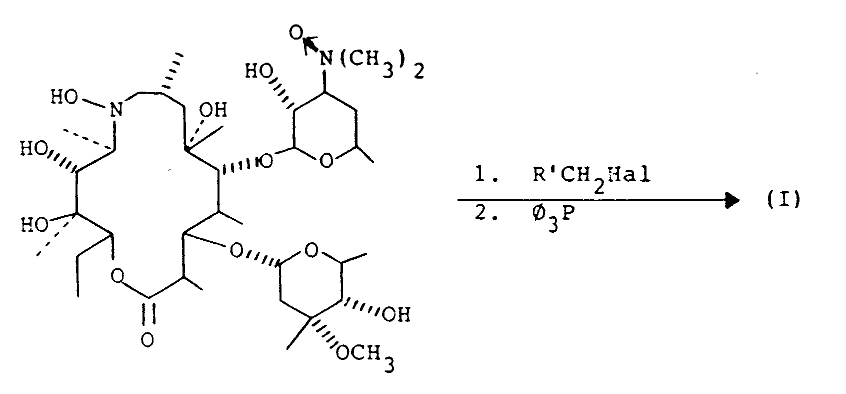
wherein R' is CH₂=CH- and Hal is a halogen, preferably bromo.

The reaction comprises the initial alkylation of the corresponding 9-deoxo-9a-aza-9a-hydroxy-9a-homoerythromycin A, 3'-N-oxide with an allyl halide, preferably bromide. In practice the reaction is carried out in a water-free reaction-inert solvent, preferably chloroform, at ambient temperatures.

Other solvents, including halogenated hydrocarbons, esters, ethers or aromatic solvents, can be employed with equally good results. In addtion, the temperature can be lowered or elevated without changing the course of the reaction, and will result in a slowing or accelerating of the reaction course, respectively.

The by-product of the alkylation, a hydrogen halide, is removed by the addition of a large excess of an inorganic base such as potassium carbonate, sodium carbonate, calcium carbonate and the like. In general, a four-fold molar excess base is adequate.

At ambient temperatures the reaction is complete in about 18-24 hours. As mentioned previously, this reaction time is a function of reaction temperature and can be adjusted accordingly.

The next step in providing the compounds of the present invention comprises deoxygenation of the 3′-N-oxide. Generally, the purity of the alkylated N-oxide is sufficiently good to allow its use directly in the next deoxygenation step; if further purification is required, the alkylated N-oxide can be passed through a silica gel column.

The deoxygenation step is carried out with an equimolar amount of triphenylphosphine plus a small excess. The reaction is conducted in a water-free reaction-inert solvent such as those employed for the alkylation reaction. The preferred solvent is tetrahydrofuran.

The reaction time can vary according to the reflux temperature of the reaction solvent. When refluxing tetrahydrofuran is employed, the reaction is complete in about 4-5 hours.

The product is isolated by removing the solvent and extracting the basic product from unwanted non-basic impurities by partitioning the residue between a water-immiscible solvent and water adjusted to a pH of about 4.5. The aqueous layer containing the product is then made basic (pH 10) and the product, as the free base, extracted with a solvent such as ethyl acetate. Further purification can be achieved by normal methods such as recrystallization or chromatography.

Acid addition salts of the compounds of this invention are readily prepared by treating compounds having formula (I) with at least an equimolar amount of the appropriate acid in a reaction-inert solvent or, in the case of the hydrochloride salts, with pyridinium hydrochloride. Since more than one basic group is present in a compound of formula (I), the addition of sufficient acid to satisfy each basic group permits formation of polyacid addition salts. The acid addition salts are recovered by filtration if they are insoluble in the reaction-inert solvent, by precipitation by addition of a non-solvent for the acid addition salt, or by evaporation of the solvent.

A variety of Gram-positive microorganisms and certain Gram-negative microorganisms, such as those of spherical or ellipsoidal shape (cocci), are suceptible to compounds of formula (I). Their in vitro activity is readily demonstrated by in vitro tests against various microorganisms in a brain-heart infusion medium by the usual two-fold serial dilution technique. Their in vitro activity renders them useful for topical application in the form of ointments, creams and the like, for sterilization purposes, e.g. sick room utensils; and as industrial antimicrobials, for example, in water treatment, slime control, paint and wood preservation.

For in vitro use, e.g. for topical application, it will often be convenient to compound the selected product by methods well known in the pharmacist's art into lotions, salves, ointments, creams, gels or the like. For such purposes, it will generally be acceptable to employ concentrations of active ingredient of from about 0.01 percent up to about 10 percent by weight based on total composition. The dosage form is applied at the site of infection ad libitum, generally at least once a day.

Additionally, formula (I) compounds of this invention are active versus Gram-positive and certain Gram-negative microorganisms in vivo via the oral and/or parenteral routes of administration in animals, including man. Their in vivo activity is more limited as regards susceptible organisms and is determined by the usual procedure which comprises infecting mice of substantially uniform weight with the test organism and subsequently treating them orally or subcutaneously with the test compound. In practice, the mice, e.g. 10, are given an intraperitoneal inoculation of suitably diluted cultures containing approximately 1 to 10 times the LD₁₀₀ (the lowest concentration of organisms required to produce 100% deaths). Control tests are simultaneously run in which mice receive inoculum of lower dilutions as a check on possible variation in virulence of the test organism. The test compound is administered 0.5 hour post-inoculation, and is repeated 4, 24 and 48 hours later. Surviving mice are held for 4 days after the last treatment and the number of survivors is noted.

When used in vivo, these novel compounds can be administered orally or parenterally, e.g. by subcutaneous or intramuscular injection, at a dosage of from about 1 mg/kg to about 200 mg/kg of body weight per day. The favored dosage range is from about 5 mg/kg to about 100 mg/kg of body weight per day and the preferred range from about 5 mg/kg to about 50 mg/kg of body weight per day. Vehicles suitable for parenteral injection may be either aqueous such as water; isotonic saline, isotonic dextrose, Ringer's solution or non-aqueous such as fatty oils of vegetable origin (cotton seed, peanut oil, corn, sesame), dimethylsulfoxide and other non-aqueous vehicles which will not interfere with therapeutic efficiency of the preparation and are non-toxic in the volume or proportion used (glycerol, propylene glycol, sorbitol). Additionally, compositions suitable for extemporaneous preparation of solutions prior to administration may advantageously be made. Such compositions may include liquid diluents; for example, propylene glycol, diethyl carbonate, glycerol, sorbitol, etc.; buffering agents, hyaluronidase, local anesthetics and inorganic salts to afford desirable pharmacological properties. These compounds may also be combined with various pharmaceutically acceptable inert carriers including solid diluents, aqueous vehicles, non-toxic organic solvents in the form of capsules, tablets, lozenges, troches, dry mixes, suspensions, solutions, elixirs and parenteral solutions or suspensions. In general, the compounds are used in various dosage forms at concentration levels ranging from about 0.5 percent to about 90 percent by weight of the total composition.

### EXAMPLE 1

### 9-Deoxo-9a-aza-9a-hydroxy-9a-homoerythromycin A 3′-N-oxide

To a solution of 9-deoxo-9a-aza-9a-homoerythromycin A (U.S. Patent 4,328,334) (10 g, 13.6 mmol) in 40 ml of methanol, a total of 50 ml of 30% aqueous hydrogen peroxide (0.58 mol) was added dropwise while stirring over a 10 minute period. After stirring overnight at ambient temperature, the reaction mixture was poured onto a stirred slurry of ice (200 g), ethyl acetate (200 ml) and water (100 ml). Excess hydrogen peroxide was quenched by cautious dropwise addition of saturated aqueous sodium sulfite until a negative starch-iodine test was indicated. The layers were separated, and the aqueous layer was extracted twice with 200 ml portions of ethyl acetate. The three organic extracts were combined, dried (anhydrous sodium sulfate), and concentrated in vacuo to afford 2 as a colorless amorphous solid (8.6 g, 82% yield).

TLC Rf = 0.20 [methylene chloride-methanol-concentrated ammonium hydroxide = 6:1:0:1 (in volume);
¹H-NMR(60 MHz, CDCl₃)delta 3.21 [6H, s (CH₃)₂N-O], 3.39 (3H, s, 3˝-OCH₃); MS m/z 576.3654 (M - C₈H₁₆O₄N, C₂₉H₅₄O₁₀N), 418.2744 (M - C₁₆H₃₀O₇N, C₂₁H₄₀O₇N).

### EXAMPLE 2

### 9-Deoxo-9a-aza-9a-allyl-9a-homoerythromycin A

To a well-stirred mixture of 4.0 g (5.2 mmol) of the product of Example 1 in 50 ml of chloroform and 28 g (0.20 mol) of suspended anhydrous potassium carbonate, 25 g (17.9 ml, 0.21 mol) of allyl bromide in 10 ml of chloroform was added dropwise over 5 minutes. Ambient temperature stirring was continued for 18 hours. Chromatography [200 g silica gel, 230-400 mesh, elution with chloroform-isopropanol-concentrated ammonium hydroxide = 8:2:0.1 (in volume)] afforded 0.72 g (colorless foam) of alkylated substrate, sufficiently pure for the next (deoxygenation) step. The entire sample was combined with 0.93 g (3.6 mmol) of triphenylphosphine in 21 ml of tetrahydrofuran, and the resulting mixture was refluxed for 4 hours. Solvent removal in vacuo afforded an oily residue which was dissolved in 150 ml of ethyl acetate. An equal volume of water was added the the pH was adjusted to 4.5 (6N hydrochloric acid). The separated aqueous phase was extracted with several 150 ml portions of ethyl acetate. Finally, the aqueous phase was combined with an equal volume of ethyl acetate, and the pH was elevated to 10.0 (10% potassium carbonate). The phases were separated, and the aqueous was extracted twice with 100 ml of ethyl acetate. The combined final (3) ethyl acetate extracts were dried (sodium sulfate) and concentrated in vacuo to an amber foam (0.55 g). Flash chromatography [50 g silica gel, 230-400 mesh, elution with chloroform-isopropanol-concentrated ammonium hydroxide = 15:1:0.1 (in volume)] afforded 408 mg (10% yield) of 3b as a colorless amorphous solid.

TLC Rf = 0.36 [methylene chloride-methanol-concentrated ammonium hydroxide = (9:1:0.1 (in volume)];
¹³C-NMR (100 MHz, CDCl₃)delta 177.8, 136.3 and 117.1 (olefinic carbons), 103.0, 95.2, 83.9, 78.5, 78.0, 77.9, 77.7, 74.8, 74.2, 72.8, 70.9, 68.8, 65.6, 64.3, 64.2, 61.2, 53.6, 49.4, 45.0, 41.9, 41.2, 40.3, (2), 35.0, 29.0, 27.8, 26.8, 22.0, 21.6, 21.5, 21.3, 18.3, 16.5, 15.0, 11.3, 9.7, 9.6; MS m/z 774.9 (M, C₄₀H₇₄O₁₂N₂), 616.4 (M - C₈H₁₆O₂N), 599.4 (M - C₈H₁₇O₃N), 458.2 (M - C₁₆H₃₀O₅N), 442 (M - C₁₆H₃₀O₆N), 157.9 (M - C₃₂H₅₈O₁₀N).

## Claims (Claims for the following Contracting State(s): AT, BE, CH, DE, FR, GB, IT, LI, LU, NL, SE)

1. A compound of the formula 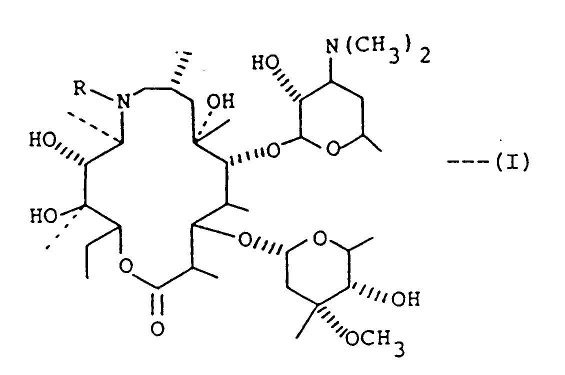 or a pharmaceutically acceptable acid addition salt thereof, wherein R is allyl

2. A pharmaceutical composition which comprises a compound as claimed in claim 1, or a pharmaceutically acceptable acid addition salt thereof, and a pharmaceutically acceptable carrier or diluent.

3. A compound of the formula (I) as claimed in claim 1, or a pharmaceutically acceptable acid addition salt thereof, for use as a medicament.

4. The use of a compound of the formula (I) as claimed in claim 1, or of a pharmaceutically acceptable acid addition salt thereof, for the manufacture of a medicament for use as an antibacterial agent.

5. A compound of the formula:- 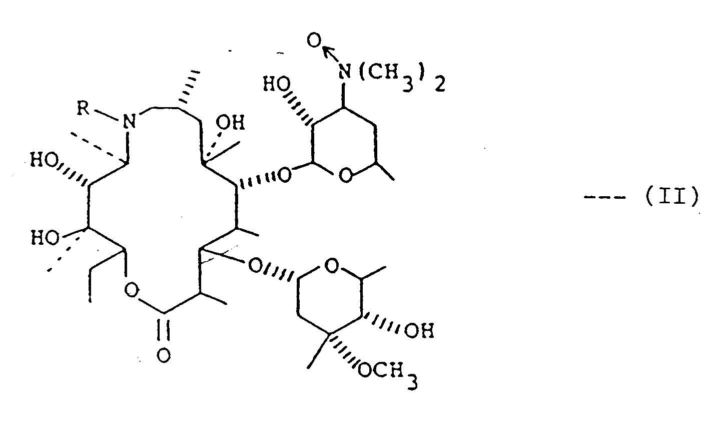 where R is hydroxy or allyl.

## Claims (Claims for the following Contracting State(s): GR)

1. A process for preparing a compound of the formula 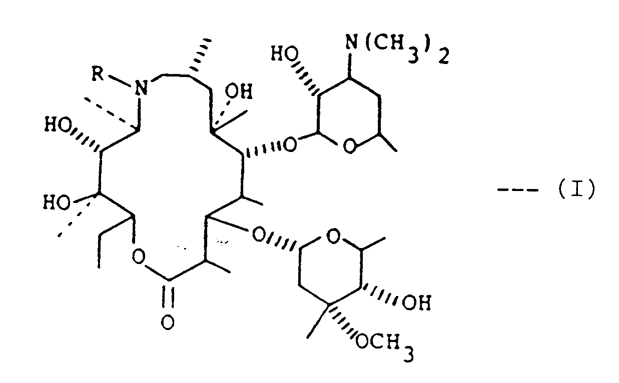 wherein R is allyl, or a pharmaceutically acceptable acid addition salt thereof, characterized by the deoxygenation of a compound of the formula 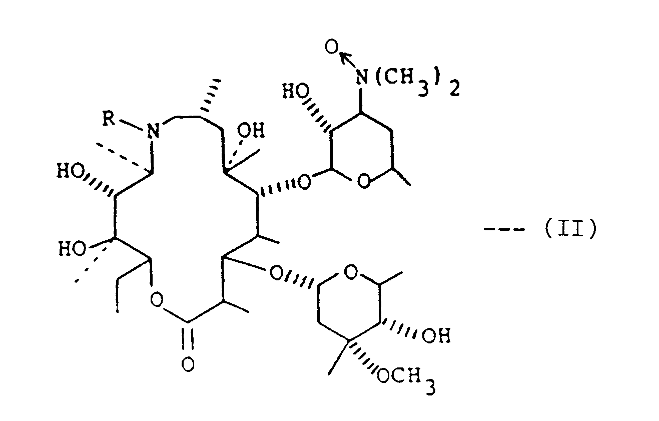 where R is as defined above,
followed by, if desired, making a pharmaceutically acceptable acid addition salt of the product.

2. The process of claim 1, wherein the deoxygenation is carried out with triphenylphosphine in a reaction-inert solvent.

3. The process of claim 2, wherein the reaction-inert solvent is tetrahydrofuran and wherein the reaction is carried out at the reflux temperature.

4. A compound having the formula (II) as set out in claim 1 in which R is hydroxy or allyl.

## Claims (Claims for the following Contracting State(s): ES)

1. A process for preparing a compound of the formula 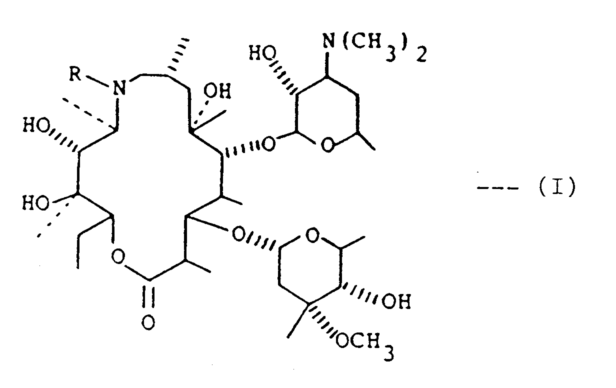 wherein R is allyl, or a pharmaceutically acceptable acid addition salt thereof, characterized by the deoxygenation of a compound of the formula 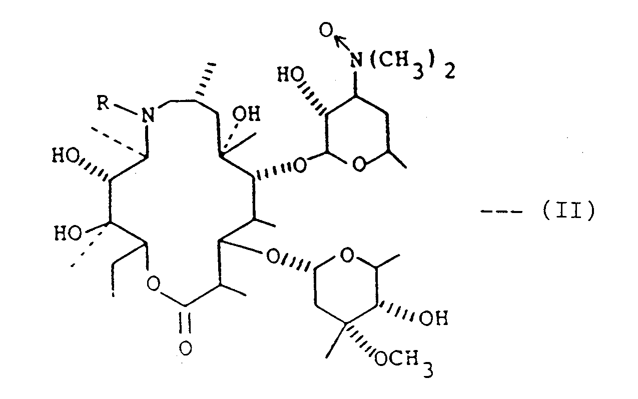 where R is as defined above,
followed by, if desired, making a pharmaceutically acceptable acid addition salt of the product.

2. The process of claim 1, wherein the deoxygenation is carried out with triphenylphosphine in a reaction-inert solvent.

3. The process of claim 2, wherein the reaction-inert solvent is tetrahydrofuran and wherein the reaction is carried out at the reflux temperature.

## Patentansprüche (Patentansprüche für folgende(n) Vertragsstaat(en): AT, BE, CH, DE, FR, GB, IT, LI, LU, NL, SE)

1. Verbindung der Formel 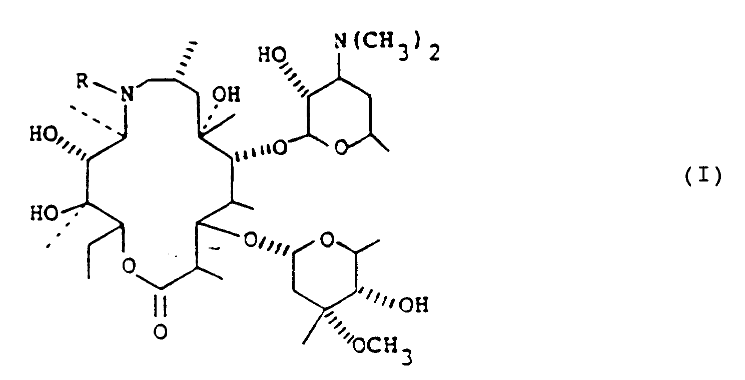 oder ein pharmazeutisch verträgliches Säureadditionssalz davon, in der R eine Allylgruppe bedeutet.

2. Verbindung nach Anspruch 1, wobei R eine Allylgruppe bedeutet.

3. Arzneimittel, umfassend eine Verbindung gemäß einem der vorangehenden Ansprüche oder ein pharmazeutisch verträgliches Salz davon und ein pharmazeutisch verträgliches Träger- oder Verdünnungsmittel.

4. Verbindung der Formel (I) gemäß einem der Ansprüche 1 und 2, oder ein pharmazeutisch verträgliches Säureadditionssalz davon, zur Verwendung als Arzneimittel.

5. Verwendung einer Verbindung der Formel (I) gemäß einem der Ansprüche 1 und 2, oder eines pharmazeutisch verträglichen Säureadditionssalzes davon, zur Herstellung eines Arzneimittels zur Verwendung als antibakterielles Mittel.

6. Verbindung der Formel: 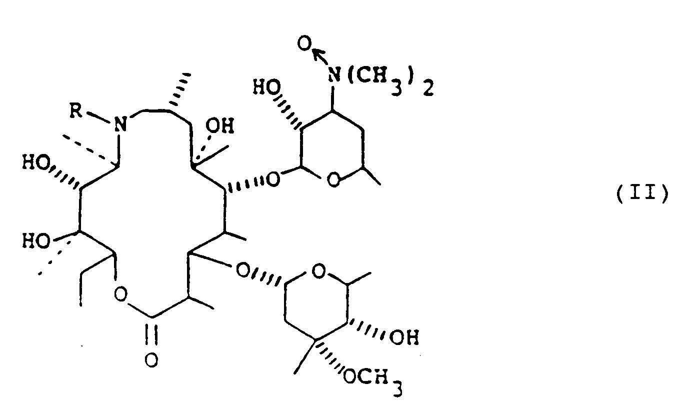 in der R eine Hydroxyl- oder Allylgruppe bedeutet.

## Patentansprüche (Patentansprüche für folgende(n) Vertragsstaat(en): GR)

1. Verfahren zur Herstellung einer Verbindung der Formel 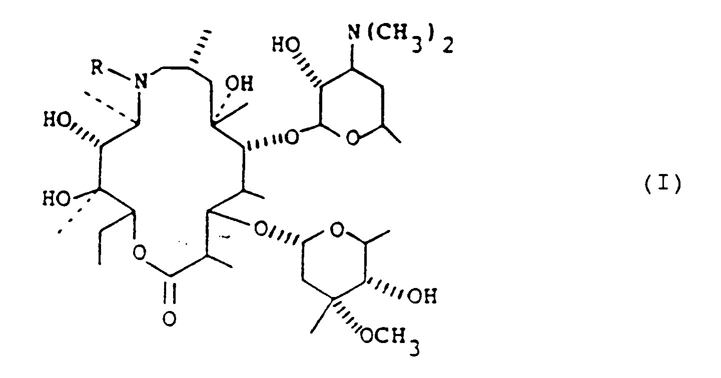 in der R eine Allylgruppe bedeutet, oder eines pharmazeutisch verträglichen Säureadditionssalzes davon, **gekennzeichnet durch** die Desoxygenierung einer Verbindung der Formel 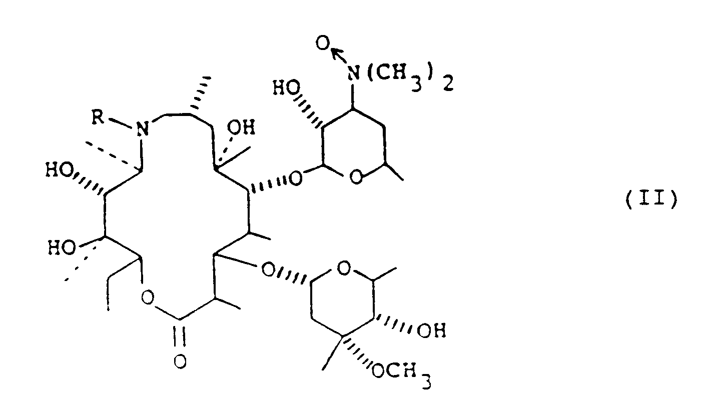 in der R wie vorstehend definiert ist, sowie durch, falls erwünscht, Herstellen eines pharmazeutisch verträglichen Säureadditionssalzes des Produkts.

2. Verfahren nach Anspruch 1, wobei die Desoxygenierung mit Triphenylphosphin in einem reaktionsinerten Lösungsmittel durchgeführt wird.

3. Verfahren nach Anspruch 2, wobei das reaktionsinerte Lösungsmittel Tetrahydrofuran ist und wobei die Umsetzung bei der Rückflußtemperatur durchgeführt wird.

4. Verbindung der Formel (II) gemäß Anspruch 1, in der R eine Hydroxyl- oder Allylgruppe bedeutet.

## Patentansprüche (Patentansprüche für folgende(n) Vertragsstaat(en): ES)

1. Verfahren zur Herstellung einer Verbindung der Formel 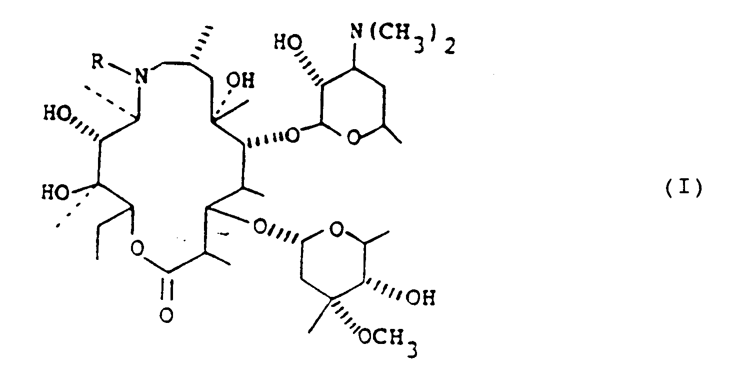 in der R eine Allylgruppe bedeutet, oder eines pharmazeutisch verträglichen Säureadditionssalzes davon, **gekennzeichnet durch** die Desoxygenierung einer Verbindung der Formel 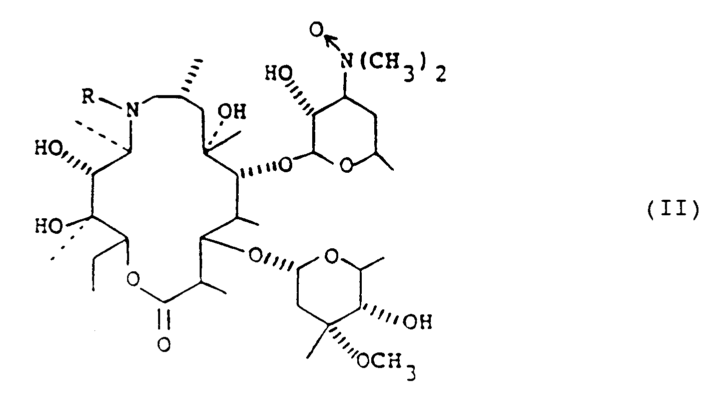 in der R wie vorstehend definiert ist, sowie durch, falls erwünscht, Herstellen eines pharmazeutisch verträglichen Säureadditionssalzes des Produkts.

2. Verfahren nach Anspruch 1, wobei die Desoxygenierung mit Triphenylphosphin in einem reaktionsinerten Lösungsmittel durchgeführt wird.

3. Verfahren nach Anspruch 2, wobei das reaktionsinerte Lösungsmittel Tetrahydrofuran ist und wobei die Umsetzung bei der Rückflußtemperatur durchgeführt wird.

## Revendications (Revendications pour l'(les) Etat(s) contractant(s) suivant(s): AT, BE, CH, DE, FR, GB, IT, LI, LU, NL, SE)

1. Composé de formule 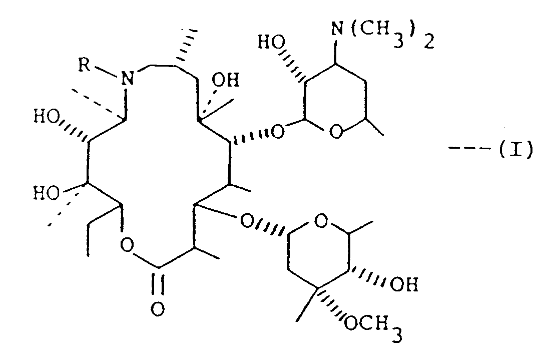 ou un de ses sels d'addition d'acides pharmaceutiquement acceptables, formule dans laquelle R représente un groupe allyle.

2. Composé suivant la revendication 1, dans lequel R représente un groupe allyle.

3. Composition pharmaceutique, qui comprend un composé suivant l'une ou l'autre des revendications précédentes, ou un de ses sels d'addition d'acides pharmaceutiquement acceptables, et un support ou diluant pharmaceutiquement acceptable.

4. Composé de formule (I) suivant l'une ou l'autre des revendications 1 et 2, ou un de ses sels d'addition d'acide pharmaceutiquement acceptables, destiné à être utilisé comme médicament.

5. Utilisation d'un composé de formule (I) suivant l'une ou l'autre des revendications 1 et 2, ou d'un de ses sels d'addition d'acides pharmaceutiquement acceptables, pour la production d'un médicament destiné à être utilisé comme agent antibactérien.

6. Composé de formule : 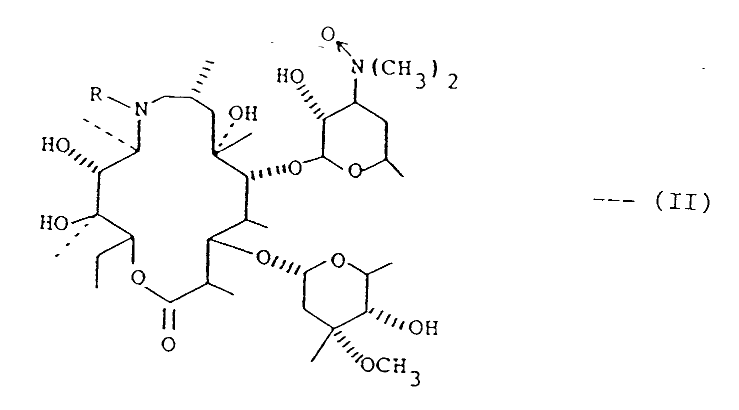 dans laquelle R représente un groupe hydroxy ou allyle.

## Revendications (Revendications pour l'(les) Etat(s) contractant(s) suivant(s): GR)

1. Procédé de préparation d'un composé de formule 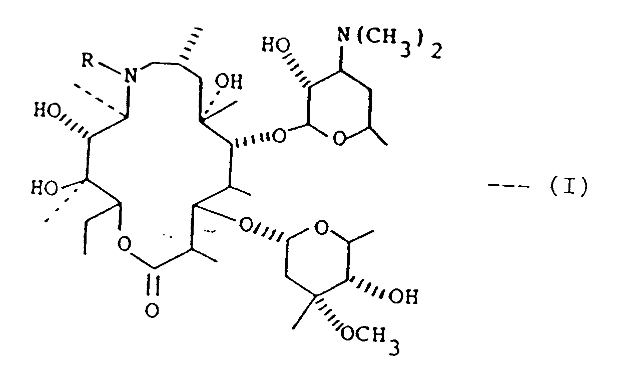 dans laquelle R représente un groupe allyle, ou d'un de ses sels d'addition d'acides pharmaceutiquement acceptables, caractérisé en ce qu'il comprend la désoxygénation d'un composé de formule 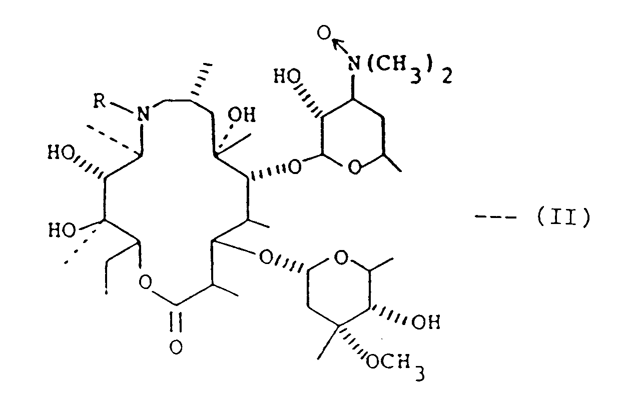 dans laquelle R répond à la définition précitée, suivie par, si cela est désiré, la préparation d'un sel d'addition d'acide pharmaceutiquement acceptable du produit.

2. Procédé suivant la revendication 1, dans lequel la désoxygénation est effectuée avec de la triphénylphosphine dans un solvant inerte vis-à-vis de la réaction.

3. Procédé suivant la revendication 2, dans lequel le solvant inerte vis-à-vis de la réaction est le tétrahydrofuranne et la réaction est conduite à la température du reflux.

4. Composé répondant à la formule (II) suivant la revendication 1, dans laquelle R représente un groupe hydroxy ou allyle.

## Revendications (Revendications pour l'(les) Etat(s) contractant(s) suivant(s): ES)

1. Procédé de préparation d'un composé de formule 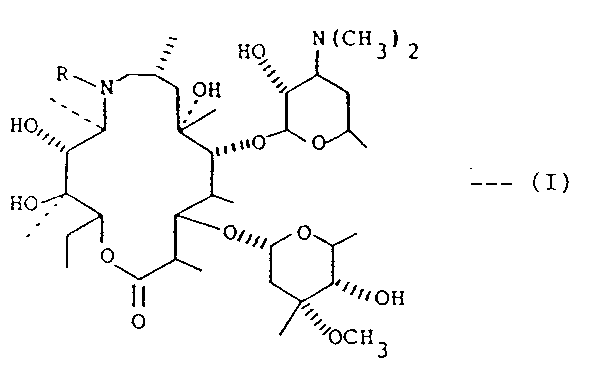 dans laquelle R représente un groupe allyle, ou d'un de ses sels d'addition d'acides pharmaceutiquement acceptables, caractérisé en ce qu'il comprend la désoxygénation d'un composé de formule 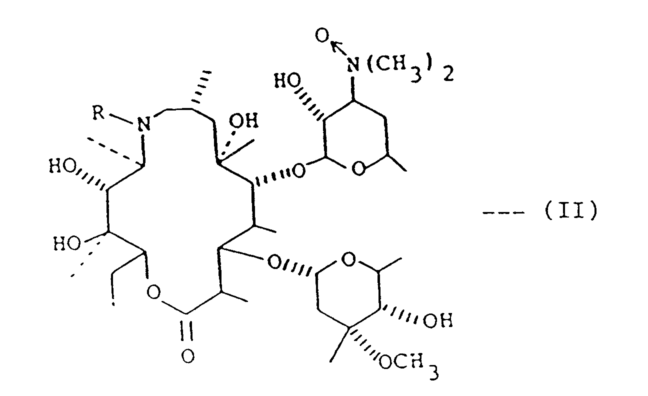 dans laquelle R répond à la définition précitée, suivie par, si cela est désiré, la préparation d'un sel d'addition d'acide pharmaceutiquement acceptable du produit.

2. Procédé suivant la revendication 1, dans lequel la désoxygénation est effectuée avec de la triphénylphosphine dans un solvant inerte vis-à-vis de la réaction.

3. Procédé suivant la revendication 2, dans lequel le solvant inerte vis-à-vis de la réaction est le tétrahydrofuranne et la réaction est conduite à la température du reflux.
